(19) Europäisches Patentamt
European Patent Office
Office européen des brevets

(11) **EP 2 876 440 B1**

(12) **FASCICULE DE BREVET EUROPEEN**

(45) Date de publication et mention de la délivrance du brevet:
**06.03.2019 Bulletin 2019/10**

(51) Int Cl.:
***G01N 33/28*** (2006.01)

(21) Numéro de dépôt: **14306834.4**

(22) Date de dépôt: **18.11.2014**

(54) **Procédé d'estimation de valeurs représentatives de répartitions du soufre et du carbone dans un produit pétrolier brut soufré**

Verfahren zur Schätzung represäntativer Werte für die Verteilung von Schwefel und Kohlenstoff in einem Rohölprodukt

Method of estimating representative values for the distribution of sulfur and carbon in a crude oil product

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.11.2013 FR 1361389**

(43) Date de publication de la demande:
**27.05.2015 Bulletin 2015/22**

(73) Titulaire: **IFP Energies nouvelles**
**92500 Rueil-Malmaison (FR)**

(72) Inventeurs:
• **Hoff, Anne**
**7036 Trondheim (NO)**
• **Lamoureux-Var, Violaine**
**78400 Chatou (FR)**
• **Meyer, Trygve**
**4020 Stavanger (NO)**
• **Pillot, Daniel**
**78100 Saint Germain en laye (FR)**

(74) Mandataire: **IFP Energies nouvelles**
**Département Propriété Industrielle**
**Rond Point de l'échangeur de Solaize**
**BP3**
**69360 Solaize (FR)**

(56) Documents cités:
**WO-A1-2010/049609    FR-A1- 2 722 296**

• LORANT F ET AL: "Characterization of sulfur in reservoir rocks by rock-eval analysis", 13TH EUROPEAN SYMPOSIUM ON IMPROVED OIL RECOVERY 2005 - 2005EUROPEAN ASSOCIATION OF GEOSCIENTISTS AND ENGINEERS,, 1 janvier 2005 (2005-01-01), pages 1-8, XP009178226, ISBN: 978-1-62276-890-5
• F. BEHAR ET AL: "Rock-Eval 6 Technology: Performances and Developments", OIL & GAS SCIENCE AND TECHNOLOGY - REVUE DE L'INSTITUT FRANÇAIS DU PÉTROLE, vol. 56, no. 2, 1 mars 2001 (2001-03-01), pages 111-134, XP055113461, ISSN: 1294-4475, DOI: 10.2516/ogst:2001013

**Description**

Le domaine de l'invention concerne une méthode mettant en oeuvre un appareil Rock-Eval™ (IFPEN) comportant un dispositif pour caractériser quantitativement le soufre (Rock-Eval Soufre). A partir de l'analyse d'un échantillon de quelques milligrammes de produit pétrolier brut soufré, cette méthode permet d'estimer les valeurs de: - la répartition du soufre et du carbone dans le résidu atmosphérique et le résidu sous vide, et - la richesse en soufre du coke rapportée au carbone du coke, à la suite de la distillation de ce produit pétrolier brut.

Cette méthode est particulièrement destinée au domaine du raffinage des produits pétroliers.

Art antérieur

On connait notamment le document WO2010/049609 qui décrit la méthode et le procédé Rock-Eval pour l'analyse du soufre. Le Rock-Eval est un dispositif comprenant au moins un four de pyrolyse en atmosphère inerte et au moins un four d'oxydation. Un module de mesure du soufre est en particulier ajouté à ce dispositif pour l'analyse du soufre.

L'art antérieur s'appliquant au raffinage pour estimer la teneur en soufre et en carbone des résidus de distillation peut être notamment:

    a. Le soufre total dans les pétroles bruts et dans les fractions de distillation de pétrole brut est conventionnellement mesuré par les méthodes ASTM D4294 (Fluorescence RX) et ASTM D5453 (Fluorescence UV).
    b. Le carbone dans les pétroles bruts et dans les fractions de distillation de pétrole brut est mesuré par la méthode ASTM D5291.
    c. Le potentiel en coke des résidus est mesuré par les méthodes ASTM D189 (Conradson Carbon Residue - CCR) et ASTM D4530 (Micro Carbon Residue - MCR).

Le coke, en tant que tel, n'est pas présent dans le pétrole brut avant le traitement de ce dernier par craquage thermique et n'est par conséquent pas mesuré dans le brut. Cependant, le potentiel à générer du coke varie d'un brut à l'autre et peut être mesuré selon les méthodes conventionnelles qui sont relativement complexes et longues. La présente invention permet de pallier à ces inconvénients.

Résumé de l'invention:

L'invention concerne un procédé d'estimation selon la revendication 1.

Ainsi, à l'aide de mesures simples de type Rock-Eval, les raffineurs peuvent obtenir rapidement des informations utiles sur la distribution du soufre entre les produits pyrolysableset le coke issus de différents types de produits pétroliers..

L'invention sera mieux comprise et ses avantages apparaîtront plus clairement à la lecture de la description qui suit et des exemples, nullement limitatifs, et illustrés par les figures ci-après annexées, parmi lesquelles :

-   les figures la et 1b donnent les mesures des composés libérés durant la pyrolyse d'un produit pétrolier soufré, avec le Rock-Eval muni d'un dispositif pour analyser le soufre (tel que décrit dans le document WO 2010/049609), en fonction de la température de l'échantillon (axe vertical à droite) et du temps de chauffe (axe horizontal):

    -   figure la : concerne les produits organiques mesurés par un détecteur à ionisation de flamme (FID),
    -   figure 1b : concerne les $SO_2$ mesuré par un détecteur UV après oxydation des effluents de pyrolyse.

-   les figures 2a, 2b, 2c donnent les mesures des composés libérés durant l'oxydation du résidu de pyrolyse d'un produit pétrolier soufré, avec le Rock-Eval Soufre (tel que décrit dans le document WO 2010/049609), en fonction de la température de l'échantillon (axe vertical à droite) et du temps de chauffe (axe horizontal):

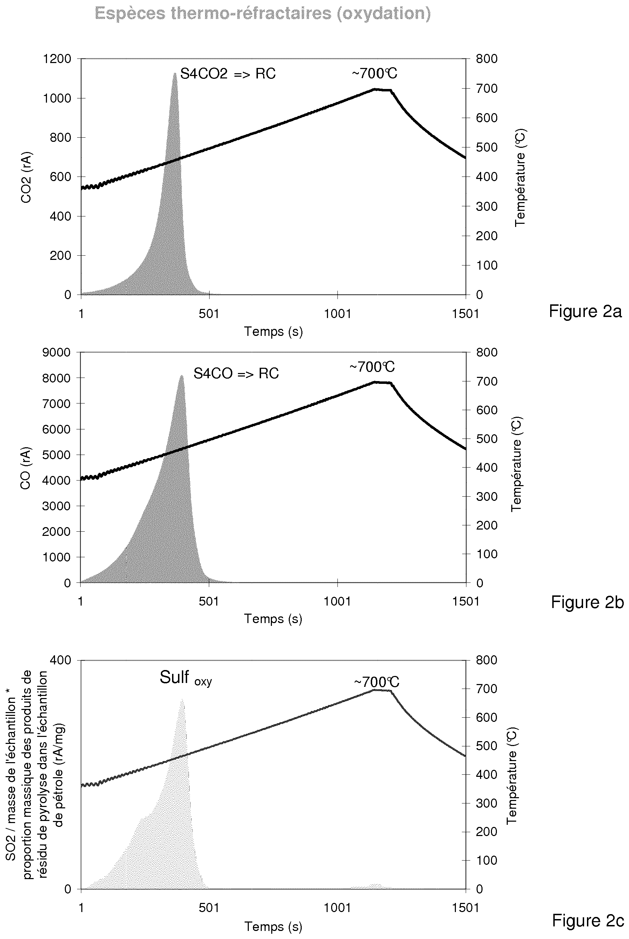

    -   figure 2a : $CO_2$ mesuré par un détecteur UV,
    -   figure 2b : CO mesuré par un détecteur UV,
    -   figure 2c : $SO_2$ mesuré par un détecteur UV,

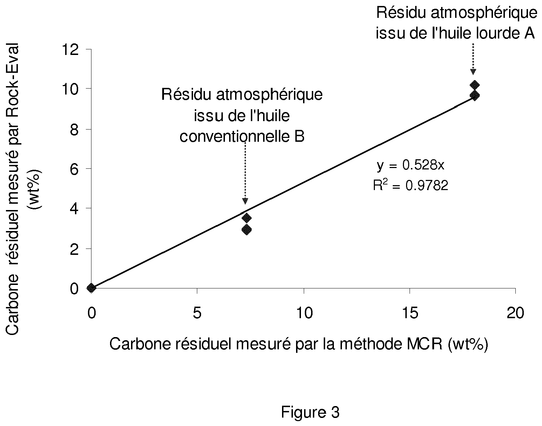

-   la figure 3 donne le taux de carbone mesuré par Rock-Eval sur un résidu de pyrolyse (en ordonnée) en fonction du taux de carbone mesuré par la méthode conventionnelle de mesure du résidu de carbone Micro Carbon Residue - MCR, ASTM D4530 (en abscisse),

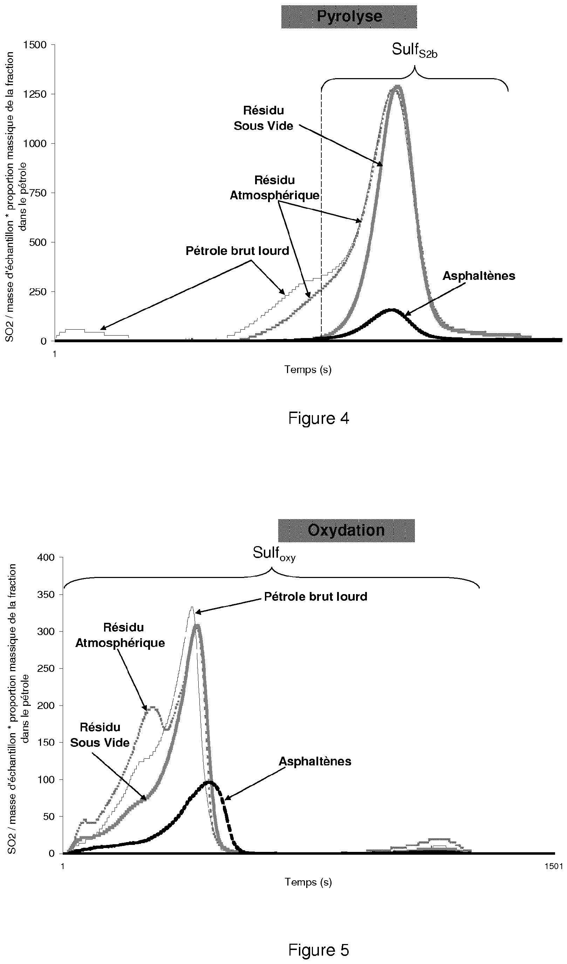

-   la figure 4 donne le signal $SO_2$ au cours de la pyrolyse de l'huile lourde A, de son résidu atmosphérique, de son résidu sous vide et de ses asphaltènes,

- la figure 5 donne le signal SO$_2$ au cours de l'oxydation de l'huile lourde A, de son résidu atmosphérique, de son résidu sous vide et de ses asphaltènes,

**Objet de l'invention**

Dans cette méthode, l'analyse d'un échantillon de produit pétrolier est réalisée avec le dispositif Rock-Eval muni d'un dispositif pour mesurer le soufre. L'ensemble du dispositif est décrit en détail dans le brevet référencé WO2010/049609.

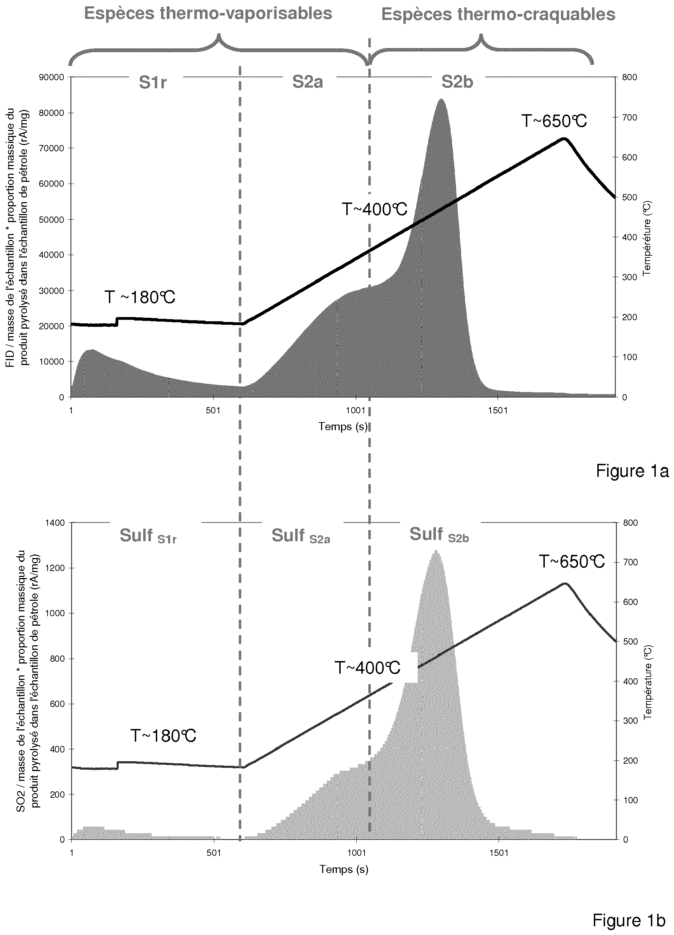

Dans cette méthode, selon le type de produit analysé, entre 3mg et 15mg de charge sont requis par analyse. L'échantillon est déposé dans une nacelle du Rock-Eval entre deux couches de silice finement broyées. L'analyse est réalisée en deux étapes automatisées. La première étape, appelée pyrolyse, consiste à chauffer l'échantillon de produit pétrolier sous un flux continu de gaz inerte, qui peut être de l'azote. La température de l'échantillon augmente d'une température initiale comprise entre 100°C et 180°C à une température finale comprise entre 650°C et 800°C, en suivant un programme de température prédéfini. Pendant cette première étape, l'échantillon de produit pétrolier libère des effluents carbonés et soufrés qui sont entraînés par le flux de gaz inerte vers des analyseurs spécifiques où ils sont mesurés en continu. La figure 1a montre les pics mesurés, de façon connue, S1r, S2a, S2b qui caractérisent les composés hydrocarbonés. Les pics S1r et S2a sont issus de la thermo-vaporisation du produit pétrolier, tandis que le pic S2b est issu du craquage thermique du produit pétrolier.

Dans le même temps, une fraction des effluents de pyrolyse entre dans un four d'oxydation. Le soufre contenu dans ces effluents est alors oxydé en SO$_2$ et est entraîné vers l'analyseur spécifique pour être mesuré en continu. Un exemple de résultat de cette mesure est montré sur la figure 1b.

A la fin de la pyrolyse, il reste au fond de la nacelle un résidu du produit pétrolier, que l'on appelle résidu de pyrolyse. Ce résidu est transféré dans sa nacelle vers un four d'oxydation. La seconde étape de l'analyse est appelée oxydation et consiste à chauffer le résidu de pyrolyse sous un flux continu d'air selon un programme de température prédéfini. La température initiale est comprise entre 300°C et 400°C, et la température finale est comprise entre 700°C et 1200°C selon le type de produit analysé.

Pendant cette deuxième étape, le résidu de pyrolyse de l'échantillon est oxydé et libère des gaz carbonés et soufrés, qui sont entraînés par le flux d'air vers les analyseurs spécifiques où ils sont mesurés en continu. Un exemple de résultat de ces mesures est montré en Figures 2a, b, et c: en (a) la mesure du CO2, en (b) la mesure du CO, et en (c) la mesure du SO2. On déduit de cette analyse différents paramètres de base caractérisant le soufre et le carbone du produit pétrolier, en particulier Sulf$_{oxy}$ et RC. Dans cette méthode, la calibration du Rock-Eval est effectuée au préalable à l'aide de gaz standards contenant du CO, du CO$_2$ et du SO$_2$ et produits pétroliers de référence dont on connaît la teneur en éléments carbone et soufre.

Parmi les paramètres de base fournis par le Rock-Eval Soufre, quatre paramètres sont utilisés dans cette méthode:

- **S2b** définit la proportion massique de composés pyrolysables lourds dans l'échantillon de produit pétrolier.

C'est la masse de composés organiques libérés par l'échantillon par craquage thermique pendant la phase de pyrolyse entre 400°C environ et la température finale de pyrolyse (Figure 1a), rapportée à la masse initiale de l'échantillon de produit pétrolier. Ces composés organiques sont les composés les plus lourds de la fraction pyrolysable de l'huile. A partir de ce paramètre, on peut estimer la proportion massique de carbone dans les composés pyrolysables lourds, en supposant que la teneur massique en carbone de ces composés est de 83%.

S2b est généralement donné dans l'unité suivante :' g (composés organiques) / 1000g (échantillon).

- **Sulf$_{S2b}$** définit la proportion massique de soufre dans les composés pyrolysables lourds de l'échantillon de produit pétrolier.

C'est la masse de soufre émise par l'échantillon au cours du craquage thermique pendant la phase de pyrolyse qui se produit entre 400°C environ et la température finale de pyrolyse (Figure 1b), simultanément à la libération des produit organiques du pic S2b, et rapportée à la masse initiale de l'échantillon de produit pétrolier .

Sulf$_{S2b}$ est généralement donné dans l'unité suivante : g (Soufre)/ 100g (échantillon).

- **RC** (residual carbon) définit la proportion massique de carbone du résidu de pyrolyse dans l'échantillon de produit pétrolier.

C'est la masse de carbone dans l'échantillon résiduel après la pyrolyse, rapportée à la masse initiale de l'échantillon de produit pétrolier; ce carbone est libéré pendant la phase d'oxydation du résidu de pyrolyse, entre 350°C et la température maximale d'oxydation qui peut varier entre 700 et 1200°C selon le programme de température choisi (Figures 2a et 2b). RC est calculé à partir des mesures de S4CO$_2$ et S4CO selon par exemple la formule suivante :

$$RC = S4CO_2 \text{ x } 12/440 + S4CO \text{ x } 12/280,$$

où :

◦ S4CO$_2$ est le pic de CO$_2$ mesuré pendant la phase d'oxydation, en mg (CO$_2$)/ g (échantillon).

◦ S4CO est le pic de CO mesuré pendant la phase d'oxydation, en mg (CO)/ g (échantillon).

RC est généralement donné dans l'unité suivante : g (C)/ 100g (échantillon).

■ **Sulf$_{oxy}$** définit la proportion massique de soufre dans le résidu de pyrolyse de l'échantillon de produit pétrolier.

C'est la masse de soufre résiduel après pyrolyse rapportée à la masse d'échantillon initial; ce soufre est libéré pendant l'oxydation du résidu de pyrolyse, entre 350°C et et la température maximale d'oxydation (Figure 2c), simultanément à la libération du carbone résiduel RC.

Sulf$_{oxy}$ est généralement donné dans l'unité suivante : g (Soufre)/ 100g (échantillon).

Les composés organiques qui sont libérés au cours de la pyrolyse entre 400°C environ et la température finale de pyrolyse, et qui sont caractérisés par les paramètres S2b (pour le carbone) et Sulf$_{S2b}$ (pour le soufre), sont issus de la fraction lourde pyrolysable du produit pétrolier analysé, qui est thermiquement craquable.

Les composés organiques qui sont libérés au cours de l'oxydation du résidu de pyrolyse, et qui sont caractérisés par les paramètres RC (pour le carbone) et Sulf$_{oxy}$ (pour le soufre), sont issus de la fraction lourde non pyrolysable du produit pétrolier analysé.

On a noté que cette fraction lourde non pyrolysable définie par l'analyse Rock-Eval apparaît être représentative du coke obtenu après raffinage d'une charge pétrolière. Ceci est soutenu par la corrélation que l'on observe entre le paramètre RC (carbone résiduel) du Rock-Eval et la teneur en carbone du résidu obtenu par ASTM D189 (CCR) et ASTM D4530 (MCR), ce résidu lui-même représentatif du coke obtenu par raffinage d'un produit pétrolier (Figure 3).

A partir de cette conception de l'origine des effluents de pyrolyse et d'oxydation du Rock-Eval, on peut définir quatre indicateurs décrivant la proportion de soufre et de carbone dans la fraction lourde pyrolysable (PHF=Pyrolyzable Heavy Fraction) et dans le coke.

Deux indicateurs décrivent la proportion de soufre dans la fraction lourde pyrolysable (Pyrolyzable Heavy Fraction : PHF) et dans le coke :

a. $S^{PHF}$= Sulf$_{S2b}$ / [Sulf$_{S2b}$ + Sulf$_{oxy}$],     exprimé en g/g
b. $S^{COKE}$= Sulf$_{oxy}$ / [Sulf$_{S2b}$ + Sulf$_{oxy}$],     exprimé en g/g.

Ils vérifient l'égalité suivante:

$$S^{PHF} + S^{COKE} = 1$$

Deux indicateurs décrivent la proportion de carbone dans la fraction lourde pyrolysable (Pyrolyzable Heavy Fraction : PHF) et dans le coke:

c. $C^{PHF}$= S2b*0.083 / [S2b*0.083 + RC],     exprimé en g/g
d. $C^{COKE}$= RC/ [S2b*0.083 + RC]     exprimé en g/g

Ils vérifient l'égalité suivante:

$$C^{PHF} + C^{COKE} = 1$$

Un cinquième indicateur décrit la richesse du coke en soufre par rapport au carbone, le carbone étant l'élément principal du coke:

e. **Sulf$_{oxy}$/RC**     exprimé en g/g

Ces 5 indicateurs sont mis en oeuvre dans le cadre de la présente invention. Les intérêts et bénéfices des 5 indicateurs de l'invention pour le procédé, comparés aux méthodes existantes d'évaluation, sont:

a. Il n'est pas connu de méthode qui, en une seule analyse, distingue les composés soufrés thermiquement extractibles, de ceux thermiquement craquables et de ceux thermiquement réfractaires.

b. Il n'y a pas non plus, à notre connaissance, de méthode qui sépare simultanément les espèces carbonés extractibles, de celles thermiquement craquables et de celles thermiquement réfractaires.

## Exemples

Huit échantillons de produits pétroliers ont été analysés avec le Rock-Eval Soufre et les indicateurs S$^{PHF}$, S$^{COKE}$, C$^{PHF}$, C$^{COKE}$, Sulf$_{oxy}$/RC ont été quantifiés.
Ces échantillons sont:

- une huile lourde A et une huile conventionnelle B,
- leur résidu atmosphérique respectif, après distillation atmosphérique,
- leur résidu sous vide respectif, après distillation sous vide du résidu atmosphérique,
- leur fraction asphaltènes respective.

Les résultats obtenus sont présentés dans les tableaux 1 et 2 et dans les figures 4 et 5.

Tableau 1: Paramètres de caractérisation du carbone des échantillons par analyse Rock-Eval Soufre

|  | TOC wt% | RC wt% | S2b mg/g | C$^{PHF}$ g/g | C$^{COKE}$ g/g |
|---|---|---|---|---|---|
| Huile lourde A | 81.8 | 8.4 | 508 | 0.83 | 0.17 |
| Résidu Atmosphérique A | 81.0 | 9.9 | 608 | 0.84 | 0.16 |
| Résidu sous vide A | 76.8 | 16.0 | 724 | 0.79 | 0.21 |
| Asphaltènes A | 78.0 | 33.5 | 489 | 0.55 | 0.45 |
| Huile conventionnelle B | 82.1 | 1.9 | 243 | 0.91 | 0.09 |
| Résidu Atmosphérique B | 84.0 | 2.9 | 490 | 0.93 | 0.07 |
| Résidu sous vide B | 80.3 | 6.1 | 849 | 0.92 | 0.08 |
| Asphaltènes B | 84.6 | 39.2 | 511.9 | 0.52 | 0.48 |

Tableau 2: Paramètres de caractérisation du soufre des échantillons par analyse Rock-Eval Soufre

|  | Sulf wt% | Sulf$_{oxy}$ wt% | Sulf$_{S2b}$ wt% | S$^{PHF}$ g/g | S$^{COKE}$ g/g | Sulf$_{oxy}$/RC g/g |
|---|---|---|---|---|---|---|
| Huile lourde A | 4.8 | 0.6 | 3.4 | 0.84 | 0.16 | 0.07 |
| Résidu Atmosphérique A | 5.5 | 0.7 | 3.9 | 0.84 | 0.16 | 0.07 |
| Résidu sous vide A | 6.2 | 1.0 | 5.1 | 0.83 | 0.17 | 0.06 |
| Asphaltènes A | 8.3 | 3.5 | 4.7 | 0.57 | 0.43 | 0.11 |
| Huile conventionnelle B | 1.8 | 0.0 | 1.1 | 0.97 | 0.03 | 0.02 |
| Résidu Atmosphérique B | 2.9 | 0.1 | 1.8 | 0.96 | 0.04 | 0.03 |
| Résidu sous vide B | 3.4 | 0.2 | 3.1 | 0.95 | 0.05 | 0.03 |
| Asphaltènes B | 4.1 | 1.5 | 2.6 | 0.63 | 0.37 | 0.04 |

La figure 4 indique qu'une partie seulement des composés soufrés lourds pyrolysables de l'huile sont présents dans les résidus de distillation et dans les asphaltènes. Néanmoins, pour le cas du résidu atmosphérique, on voit sur la figure 4 qu'il contient presque autant de composés soufrés pyrolysables lourds que l'huile de départ.
La figure 5 suggère que certains composés soufrés composant les résidus de pyrolyse des résidus de distillation et des asphaltènes sont différents de ceux du résidu de pyrolyse de l'huile.

Si l'on compare les cinq indicateurs $S^{PHF}$, $S^{COKE}$, $C^{PHF}$, $C^{COKE}$, $Sulf_{oxy}/RC$ dans les tableaux 1 et 2, des huiles brutes, de leurs résidus de distillation atmosphérique et de leurs résidus de distillation sous vide, on observe des résultats très proches. Par comparaison, ces cinq indicateurs appliqués aux asphaltènes montrent des résultats très différents de ceux obtenus pour les huiles brutes et les résidus de distillation.

Cela montre clairement que les valeurs des indicateurs selon l'invention, ($S^{PHF}$, $S^{COKE}$, $C^{PHF}$, $C^{COKE}$, $Sulf_{oxy}/RC$), obtenues sur huile brute à l'aide d'une analyse Rock-Eval ($S_{2b}$, $Sulf_{S2b}$, $Sulf_{oxy}$, RC), permettent d'estimer celles des résidus de distillation. Ces estimations rapides, et donc peu coûteuses, sont très avantageuses pour évaluer la réactivité thermique du soufre et du carbone des résidus d'une charge à raffiner.

## Revendications

**1.** Procédé d'estimation, pour un produit pétrolier brut soufré, de valeurs représentatives de répartitions du soufre et du carbone dans des résidus atmosphérique et sous vide issus de distillation, et d'une valeur représentative du soufre du coke, procédé dans lequel on effectue les étapes suivantes:

- à partir d'un échantillon de quelques milligrammes dudit produit pétrolier brut soufré, on mesure au moins des paramètres S2b, $Sulf_{S2b}$, RC, $Sulf_{oxy}$ à l'aide d'un dispositif comprenant au moins un four de pyrolyse en atmosphère inerte et au moins un four d'oxydation, ledit dispositif comportant un module de mesure du soufre, S2b étant la proportion massique de composés pyrolysables lourds contenus dans ledit échantillon, $Sulf_{S2b}$ étant la proportion massique de soufre dans les composés pyrolysables lourds contenus dans ledit échantillon, RC étant la proportion massique de carbone du résidu de pyrolyse dudit échantillon, et $Sulf_{oxy}$ étant la proportion massique de soufre dans le résidu de pyrolyse dudit échantillon,

- on déduit desdites mesures desdits paramètres lesdites valeurs de répartitions en soufre et en carbone dans les résidus de distillation, et une teneur en soufre rapportée au carbone dans le coke, lesdites valeurs représentatives de répartitions du soufre et du carbone dans des résidus atmosphérique et sous vide issus de distillation étant déterminées selon :

- $S^{PHF}$= $Sulf_{S2b}$ / [$Sulf_{S2b}$ + $Sulf_{oxy}$ ], où $S^{PHF}$ correspond à la proportion de soufre dans la fraction lourde pyrolysable, avec lesdits paramètres $Sulf_{S2b}$, $Sulf_{S2b}$ et $Sulf_{oxy}$ exprimés en gramme par gramme dudit échantillon,
- $S^{COKE}$= $Sulf_{oxy}$ / [$Sulf_{S2b}$ + $Sulf_{oxy}$ ], où $S^{COKE}$ correspond à la proportion de soufre dans le coke, avec avec lesdits paramètres $Sulf_{S2b}$ , $Sulf_{S2b}$ et $Sulf_{oxy}$ exprimés en gramme par gramme dudit échantillon,
avec $S^{PHF}+S^{COKE}=1$,
- $C^{PHF}$= S2b*0.083 / [S2b*0.083 + RC], où $C^{PHF}$ correspond à la poportion de carbone dans la fraction lourde pyrolysable, avec lesdits paramètres S2b et RC exprimés en gramme par gramme dudit échantillon,
- $C^{COKE}$= RC/ [S2b*0.083 + RC], où $C^{COKE}$ correspond à la poportion de carbone dans le coke, avec lesdits paramètres S2b et RC exprimés en gramme par gramme dudit échantillon,
avec $C^{PHF}+C^{COKE} =1$ ;
et la valeur représentative de la richesse en soufre par rapport au carbone du coke étant déterminée selon :
$Sulf_{oxy} /RC$, avec lesdits paramètres $Sulf_{oxy}$ et RC exprimés en gramme par gramme dudit échantillon.

## Patentansprüche

**1.** Verfahren zum Schätzen, für ein schwefelhaltiges Rohölprodukt, der Werte, die für die Schwefel- und Kohlenstoffverteilungen in den Rückständen in der Atmosphäre und aus der Vakuumdestillation repräsentativ sind, und eines Werts, der für den Schwefel des Koks repräsentativ ist, wobei die folgenden Schritte durchgeführt werden:

- an einer Probe von einigen Milligramm des schwefelhaltigen Rohölprodukts werden mindestens die Parameter S2b, $Sulf_{S2b}$, RC, $Sulf_{oxy}$ mit Hilfe einer Vorrichtung gemessen, umfassend mindestens einen Pyrolyseofen in inerter Atmosphäre und mindestens einen Oxidationsofen, wobei die Vorrichtung ein Modul zur Messung von Schwefel umfasst, wobei S2b der Massenanteil der schweren pyrolysierbaren Verbindungen ist, die in der

Probe enthalten sind, $\text{Sulf}_{S2b}$ der Massenanteil von Schwefel in den schweren pyrolysierbaren Verbindungen ist, die in der Probe enthalten sind, RC der Massenanteil von Kohlenstoff des Pyrolyserückstands der Probe ist, und $\text{Sulf}_{oxy}$ der Massenanteil von Schwefel im Pyrolyserückstand der Probe ist,

- aus diesen Messwerten der Parameter werden die Werte der Schwefel- und Kohlenstoffverteilungen in den Destillationsrückständen und ein Gehalt an Schwefel bezogen auf Kohlenstoff im Koks abgeleitet, wobei die Werte, die für die Schwefel- und Kohlenstoffverteilungen in den Rückständen in der Atmosphäre und aus der Vakuumdestillation repräsentativ sind, bestimmt werden gemäß:

- $S^{PHF} = \text{Sulf}_{S2b}/[\text{Sulf}_{S2b} + \text{Sulf}_{oxy}]$, wobei $S^{PHF}$ der Menge an Schwefel in der pyrolysierbaren schweren Fraktion entspricht, wobei die Parameter $\text{Sulf}_{S2b}$, $\text{Sulf}_{S2b}$ und $\text{Sulf}_{oxy}$ in Gramm pro Gramm der Probe ausgedrückt sind,

- $S^{COKE} = \text{Sulf}_{oxy}/[\text{Sulf}_{S2b} + \text{Sulf}_{oxy}]$, wobei $S^{COKE}$ der Menge an Schwefel im Koks entspricht, wobei die Parameter $\text{Sulf}_{S2b}$, $\text{Sulf}_{S2b}$ und $\text{Sulf}_{oxy}$ in Gramm pro Gramm der Probe ausgedrückt sind, wobei $S^{PHF} + S^{COKE} = 1$,

- $C^{PHF} = S2b*0{,}083/[S2b*0{,}083 + RC]$, wobei $C^{PHF}$ der Menge an Kohlenstoff in der pyrolysierbaren schweren Fraktion entspricht, wobei die Parameter S2b und RC in Gramm pro Gramm der Probe ausgedrückt sind,

- $C^{COKE} = RC/[S2b*0{,}083 + RC]$, wobei $C^{COKE}$ der Menge an Kohlenstoff im Koks entspricht, wobei die Parameter S2b und RC in Gramm pro Gramm der Probe ausgedrückt sind,

wobei $C^{PHF} + C^{COKE} = 1$,

und wobei der Wert, der für die Anreicherung mit Schwefel in Bezug auf den Kohlenstoff des Koks repräsentativ ist, bestimmt wird gemäß:

$\text{Sulf}_{oxy}/RC$, wobei die Parameter $\text{Sulf}_{oxy}$ und RC in Gramm pro Gramm der Probe ausgedrückt sind.

## Claims

1. A method of estimating, for a raw sulfur-containing petroleum product, values representative of sulfur and carbon distributions in atmospheric and vacuum distillation residues, and a value representative of the sulfur in the coke, a method wherein the following stages are carried out:

- from a sample of some milligrams of said raw sulfur-containing petroleum product, measuring at least parameters S2b, $\text{Sulf}_{S2b}$, RC, $\text{Sulf}_{oxy}$ using a device comprising at least one inert atmosphere pyrolysis oven and at least one oxidation oven, said device comprising a sulfur measurement module, S2b being the mass proportion of heavy pyrolyzable compounds contained in said sample, $\text{Sulf}_{S2b}$ being the mass proportion of sulfur in the heavy pyrolyzable compounds contained in said sample, RC being the mass proportion of carbon of the pyrolysis residue of said sample, and $\text{Sulf}_{oxy}$ being the mass proportion of sulfur in the pyrolysis residue of said sample,

- deducing from said measurements of said parameters said values of the sulfur and carbon distributions in the distillation residues, and a sulfur content in relation to the carbon content in the coke, said values representative of sulfur and carbon distributions in atmospheric and vacuum distillation residues being determined as follows:

- $\mathbf{S^{PHF}} = \text{Sulf}_{S2b} / [\text{Sulf}_{S2b} + \text{Sulf}_{oxy}]$, where $\mathbf{S^{PHF}}$ corresponds to the proportion of sulfur in the pyrolyzable heavy fraction, with said parameters $\text{Sulf}_{S2b}$, $\text{Sulf}_{S2b}$ and $\text{Sulf}_{oxy}$ expressed in gram per gram of said sample,

- $\mathbf{S^{COKE}} = \text{Sulf}_{oxy} / [\text{Sulf}_{S2b} + \text{Sulf}_{oxy}]$, where $\mathbf{S^{COKE}}$ corresponds to the proportion of sulfur in the coke, with said parameters $\text{Sulf}_{S2b}$, $\text{Sulf}_{S2b}$ and $\text{Sulf}_{oxy}$ expressed in gram per gram of said sample,

with $\mathbf{S^{PHF}} + S^{COKE} = 1$,

- $\mathbf{C^{PHF}} = S2b*0.083 / [S2b*0.083 + RC]$, where $\mathbf{C^{PHF}}$ corresponds to the proportion of carbon in the pyrolyzable heavy fraction, with said parameters S2b and RC expressed in gram per gram of said sample,

- $\mathbf{C^{COKE}} = RC / [S2b*0.083 + RC]$, where $\mathbf{C^{COKE}}$ corresponds to the proportion of carbon in the coke, with said parameters S2b and RC expressed in gram per gram of said sample,

with $\mathbf{C^{PHF}} + \mathbf{C^{COKE}} = 1$;

and the value representative of the richness in sulfur in relation to the carbon content in the coke is determined as follows:

$\mathbf{Sulf_{oxy}/RC}$, with said parameters Sulfoxy and RC expressed in gram per gram of said sample.

Figure 1a

Figure 1b

Espèces thermo-réfractaires (oxydation)

Figure 2a

Figure 2b

Figure 2c

Figure 3

Figure 4

Figure 5

**EP 2 876 440 B1**

## RÉFÉRENCES CITÉES DANS LA DESCRIPTION

**Documents brevets cités dans la description**

- WO 2010049609 A